(19) Europäisches Patentamt
European Patent Office
Office européen des brevets

(11) **EP 4 729 926 A1**

(12) **EUROPEAN PATENT APPLICATION**
published in accordance with Art. 153(4) EPC

(43) Date of publication:
**22.04.2026 Bulletin 2026/17**

(21) Application number: **24823190.4**

(22) Date of filing: **22.05.2024**

(51) International Patent Classification (IPC):
**G01N 21/3577** (2014.01)    **G01N 21/552** (2014.01)
**G01N 33/32** (2006.01)

(52) Cooperative Patent Classification (CPC):
**G01N 21/3577; G01N 21/552; G01N 33/32**

(86) International application number:
**PCT/JP2024/018806**

(87) International publication number:
**WO 2024/257568 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.06.2023 JP 2023096935**

(71) Applicant: **SHIMADZU CORPORATION**
**Kyoto-shi, Kyoto 604-8511 (JP)**

(72) Inventors:
• **IIDA, Eiji**
**Kyoto-shi, Kyoto 604-8511 (JP)**
• **NAKA, Kosuke**
**Kyoto-shi, Kyoto 604-8511 (JP)**
• **MARUYAMA, Karen**
**Kyoto-shi, Kyoto 604-8511 (JP)**

(74) Representative: **Müller-Boré & Partner**
**Patentanwälte PartG mbB**
**Friedenheimer Brücke 21**
**80639 München (DE)**

(54) **METHOD FOR OBSERVING CURED STATE OF COATING MATERIAL**

(57)    A method for in-situ observation of a curing state of a paint is provided, which allows evaluation of the curing state of the paint from the viewpoint of temperature and time during the paint curing process, thereby reducing the burden on the user. The method includes: a step of bringing a sample, which is a mixture of a curing agent and a paint, into contact with a surface of a diamond prism; a step of measuring an infrared absorption spectrum of the sample while controlling a temperature of the sample, and acquiring time-series data of an amount of a functional group possessed by the curing agent in the sample from the obtained infrared absorption spectrum; and a step of visualizing a relationship between time or temperature and the amount of the functional group possessed by the curing agent in the sample.

FIG.1

$$-R-COO-R'-OH\ +\ O=C=N-R''=\ -R-COO-R'-O-\overset{\overset{\textstyle H}{|}}{\underset{\underset{\textstyle O}{||}}{C}}-N-R''$$

ACRYLIC POLYOL        ISOCYANATE        ACRYLIC URETHANE
(MAIN AGENT)        (CURING AGENT)        RESIN

## Description

[Technical Field]

The present invention relates to a method for observing a curing state of a paint.

[Background Art]

Polyisocyanates derived from aliphatic diisocyanates or alicyclic diisocyanates are known to be non-yellowing curing agents excellent in weather resistance, chemical resistance, and flexibility. Blocked polyisocyanates, in which the isocyanate groups of polyisocyanate are blocked by a thermally dissociative blocking agent, do not react at room temperature even when mixed with an active hydrogen compound as a main agent, but when heated, the blocking agent dissociates, regenerating the isocyanate groups and allowing curing to proceed.

In order for this thermal Curing paint to exhibit its intended performance, it is necessary to find appropriate curing conditions. As a method for determining these curing conditions, for example, Kanji Mori, "Tracking Method of Crosslinking Density during Curing Process of Paint Film -Proportionality between Storage Modulus and Crosslinking Density-", J. Jpn. Soc. Colour Mater., 86(4), 123-127 (2013) (Non-Patent Literature 1) discloses a pendulum-type viscoelasticity measuring device that measures viscoelasticity reflecting changes in crosslinking density closely related to curing, or a method of analyzing changes in the abundance and reaction rate of isocyanate by infrared spectroscopic analysis.

Furthermore, various countries and regions are implementing various initiatives to achieve carbon neutrality by 2050, such as introducing renewable energy and reducing $CO_2$ emissions (the Chinese government plans to achieve zero by 2060). Focusing particularly on the automotive industry, which has high $CO_2$ emissions, it is reported that about 25% of $CO_2$ emissions in the manufacturing process originate from the paint coating process. Automotive manufacturers and paint manufacturers are advancing the development of new paints aimed at reducing $CO_2$ in this process. In the coating process, after the paint is applied, it is baked and dried at high temperatures to thermally cure the paint. Shortening the curing time and curing at low temperatures are said to be effective in reducing $CO_2$ emissions.

[Prior Art Literature]

[Non-Patent Literature]

[Non-Patent Literature 1] Kanji Mori, "Tracking Method of Crosslinking Density during Curing Process of Paint Film -Proportionality between Storage Modulus and Crosslinking Density-", J. Jpn. Soc. Colour Mater., 86(4), 123-127 (2013)
[Non-Patent Literature 2] Morio Tsuge, "Application of Fourier Transform Infrared Spectroscopy to the Analysis of Thermal Curing Resins", Thermal Curing Resins, Vol. 12 No. 4 (1991), p209-234

[Summary of Invention]

[Problem to be Solved by the Invention]

In the method of analysis by infrared spectroscopy described in Non-Patent Literature 1, the curing process at a predetermined temperature (140°C) is evaluated using a mixture of a hydroxyl group-containing acrylic resin and an isocyanate curing agent as a model paint. However, while this method can evaluate curing changes over time, it cannot evaluate the curing process due to temperature changes. To find the optimal curing temperature in paint development, the method described in Non-Patent Literature 1 requires the user to set the temperature each time to acquire analysis data, and also requires manually compiling the data acquired for each temperature, thus demanding excessive labor to find the optimal curing conditions.

The present invention has been made to solve the above problems, and an object thereof is to provide a method that allows evaluation of the curing state of a paint from the viewpoint of temperature and time during the paint curing process, thereby reducing the burden on the user.

[Means for Solving the Problem]

A first aspect of the present invention relates to a method for in-situ observation of a curing state of a paint, comprising: a step of bringing a sample, which is a mixture of a curing agent and a paint, into contact with a surface of a diamond prism; a step of measuring an infrared absorption spectrum of the sample while controlling a temperature of the sample, and acquiring time-series data of an amount of a functional group possessed by the curing agent in the sample from the obtained infrared absorption spectrum; and a step of visualizing a relationship between time or temperature and the amount of the functional group possessed by the curing agent in the sample.

[Effects of Invention]

According to the method of the present invention, data regarding the curing state of the paint can be acquired from the viewpoint of temperature and time during the paint curing process, thereby reducing the user's burden required to find the curing conditions for the paint to be evaluated.

[Brief Description of Drawings]

[FIG. 1] FIG. 1 is a diagram showing a reaction formula of an acrylic urethane resin used in Experimental Example 1.
[FIG. 2] FIG. 2 shows spectra of the paint before and after curing in Experimental Example 1.
[FIG. 3] FIG. 3 is an enlarged spectrum of a portion from 3100 to 2150 cm$^{-1}$ in FIG. 2.
[FIG. 4] FIG. 4 is a graph showing a relationship between reaction rate and thermal Curing time at each constant temperature.

[Description of Embodiments]

A first aspect of the present invention is a method for in-situ observation of a curing state of a paint, comprising: a step of bringing a sample, which is a mixture of a curing agent and a paint, into contact with a surface of a diamond prism; a step of measuring an infrared absorption spectrum (IR spectrum: InfraRed absorption spectrometry) of the sample while controlling a temperature of the sample, and acquiring time-series data of an amount of a functional group possessed by the curing agent in the sample from the obtained infrared absorption spectrum; and a step of visualizing a relationship between time or temperature and the amount of the functional group possessed by the curing agent in the sample.

The first aspect of the present invention presupposes the use of an apparatus equipped with a diamond prism, capable of measuring infrared absorption spectra while changing the temperature of the sample. Specific examples of such an apparatus include a heating-type single-reflection attenuated total reflection measuring device MicromATR (manufactured by Czitech) combined with a Fourier Transform Infrared Spectrophotometer (FTIR) IRTracer-100 $^{(trademark)}$ (manufactured by Shimadzu Corporation), and a Golden Gate $^{(tradentark)}$ (diamond ATR (Attenuated Total Reflection) device) (manufactured by Specac) combined with a 4000 series $^{(trademark)}$ temperature controller (manufactured by Specac). However, the apparatus is not limited thereto as long as it satisfies the aforementioned prerequisite.

In the first aspect of the present invention, first, a sample, which is a mixture of a curing agent and a paint, is brought into contact with the surface of the diamond prism. By thus directly applying the sample to the surface of the diamond prism, data from contaminants do not enter the infrared absorption spectrum.

In the first aspect of the present invention, an infrared absorption spectrum of the sample is measured while controlling the temperature of the sample, and time-series data of the amount of the functional group possessed by the curing agent in the sample is acquired from the obtained infrared absorption spectrum. Thus, in the first aspect of the present invention, the curing process of the paint is observed in-situ, and data regarding the curing state of the paint is acquired from the viewpoint of temperature and time.

In the first aspect of the present invention, the measurement of the infrared absorption spectrum of the sample while controlling the temperature of the sample preferably includes both measuring the infrared absorption spectrum of the sample while maintaining the temperature of the sample constant, and measuring the infrared absorption spectrum of the sample while changing the temperature of the sample at a predetermined rate of change. By measuring the infrared absorption spectrum of the sample while maintaining the temperature of the sample constant, time-series data showing the relationship between time and the amount of the functional group possessed by the curing agent in the sample can be obtained. On the other hand, by measuring the infrared absorption spectrum of the sample while changing the temperature of the sample at a predetermined rate of change, time-series data showing the relationship between temperature and the amount of the functional group possessed by the curing agent in the sample can be obtained. The temperature to be maintained constant is not particularly limited and can be appropriately selected according to the temperature at which the curing agent contained in the sample cures. Further, the predetermined rate of change when changing the temperature is also not particularly limited, and the temperature change may, of course, be continuous or intermittent.

In the first aspect of the present invention, next, the relationship between time or temperature and the amount of the functional group possessed by the curing agent in the sample is visualized. Specifically, by graphing the obtained time-series data with time or temperature on the horizontal axis and the amount of the functional group possessed by the curing agent on the vertical axis, the relationship between time or temperature and the amount of the functional group possessed by the curing agent is visualized. Thus, in the first aspect of the present invention, curing changes over time are evaluated, and the curing process due to temperature changes is also evaluated. This can reduce the user's burden required to find the curing conditions for the paint to be evaluated.

Here, the functional group possessed by the curing agent is not particularly limited as long as its relationship between the infrared spectrum and curing temperature is known, such as an isocyanate group in the case of an isocyanate curing agent. Examples of such functional groups possessed by the curing agent include an epoxy group, a primary amino group, a secondary amino group, a primary amide group, a tertiary amide group, an ether group, a hydroxyl group, a carbonyl group, and the like, as described in, for example, Morio Tsuge, "Application of Fourier Transform Infrared Spectroscopy to the Analysis of Thermal Curing Resins", Thermal Curing Resins, Vol. 12 No. 4 (1991), p209-234 (Non-Patent Literature 2).

In the first aspect of the present invention, as the curing agent, a known curing agent generally used for curing a thermal Curing resin can be used, but it is preferable that the curing agent is an isocyanate curing agent and the functional group possessed by the curing agent in the sample is an isocyanate group. When an isocyanate curing agent is used, a polyfunctional isocyanate curing agent such as an aliphatic, alicyclic, aromatic group-containing aliphatic, or aromatic diisocyanate, a dimer of diisocyanate, a trimer of diisocyanate (preferably an isocyanurate-type isocyanate (so-called isocyanurate)), or the like can be used.

Representative isocyanate curing agents include, for example, aromatic diisocyanates such as tolylene diisocyanate (TDI), 4,4'-diphenylmethane diisocyanate (MDI), xylylene diisocyanate (XDI), and metaxylylene diisocyanate (MXDI); aliphatic diisocyanates such as hexamethylene diisocyanate (HDI), tetramethylene diisocyanate, 2-methyl-pentane-1,5-diisocyanate, 3-methyl-pentane-1,5-diisocyanate, lysine diisocyanate, and trioxyethylene diisocyanate; alicyclic diisocyanates such as isophorone diisocyanate (IPDI), cyclohexyl diisocyanate, 4,4'-dicyclohexylmethane diisocyanate, norbornane diisocyanate, hydrogenated tolylene diisocyanate, hydrogenated xylene diisocyanate, and hydrogenated tetramethylxylene diisocyanate; and their biuret bodies, nurate bodies, trimethylolpropane (TMP) adduct bodies, uretdione bodies, and the like. These may be used alone or in combination of two or more.

As the isocyanate curing agent, a blocked isocyanate can also be used. The blocked isocyanate can be prepared by blocking the isocyanate groups of the polyfunctional isocyanate curing agent described above with a blocking agent. The blocking agent is one that adds to the polyisocyanate group, is stable at room temperature, but can regenerate free isocyanate groups when heated to or above the dissociation temperature. As the blocking agent, commonly used ones such as ε-caprolactam and butyl cellosolve can be used.

A suitable specific example of the isocyanate curing agent includes, for example, Duranate <sup>(trademark)</sup> TPA-100 (manufactured by Asahi Kasei Corporation), which is disclosed in Non-Patent Literature 1 and whose main component is a trimer (isocyanurate) of hexamethylene diisocyanate.

As the paint (main agent) contained in the sample used in the first aspect of the present invention, conventionally known appropriate paints used in combination with an isocyanate curing agent can be used without particular limitation, but a suitable example includes a hydroxyl group-containing acrylic resin.

The hydroxyl group-containing acrylic resin can be suitably prepared by copolymerizing a monomer mixture containing a hydroxyl group-containing ethylenically unsaturated monomer. Examples of the hydroxyl group-containing ethylenically unsaturated monomer include 2-hydroxyethyl (meth)acrylate, hydroxypropyl (meth)acrylate, 2,3-dihydroxybutyl (meth)acrylate, 4-hydroxybutyl (meth)acrylate, reaction products of these hydroxyl group-containing (meth)acrylates and ε-caprolactone, and esterified products of polyhydric alcohols such as polyethylene glycol mono(meth)acrylate with acrylic acid or methacrylic acid. Furthermore, a reaction product obtained by ring-opening polymerization of ε-caprolactone with a monoesterified product of the above polyhydric alcohol and acrylic acid or methacrylic acid can also be used.

The monomer mixture may contain ethylenically unsaturated monomers other than the hydroxyl group-containing ethylenically unsaturated monomer. Examples of such ethylenically unsaturated monomers include (meth)acrylic acid alkyl ester monomers such as methyl (meth)acrylate, ethyl (meth)acrylate, propyl (meth)acrylate, n-, i- or t-butyl (meth)acrylate, 2-ethylhexyl (meth)acrylate, and lauryl (meth)acrylate; carboxyl group-containing ethylenically unsaturated monomers, such as carboxyl group-containing monomers like acrylic acid, methacrylic acid, crotonic acid, maleic acid, itaconic acid, and fumaric acid, and dicarboxylic acid monoester monomers like ethyl maleate, butyl maleate, ethyl itaconate, and butyl itaconate; amino group-containing ethylenically unsaturated monomers such as allylamine and p-vinylaniline; alicyclic group-containing ethylenically unsaturated monomers such as cyclopentyl (meth)acrylate, cyclohexyl (meth)acrylate, isobornyl (meth)acrylate, tricyclodecanyl (meth)acrylate, and adamantyl (meth)acrylate; (meth)acrylic acid aminoalkyl ester monomers such as aminoethyl (meth)acrylate, dimethylaminoethyl (meth)acrylate, and butylaminoethyl (meth)acrylate; (meth)acrylic acid aminoalkylamide monomers such as aminoethyl (meth)acrylamide, dimethylaminomethyl (meth)acrylamide, and methylaminopropyl (meth)acrylamide; other amide group-containing ethylenically unsaturated monomers such as acrylamide, methacrylamide, N-methylolacrylamide, methoxybutylacrylamide, and diacetone acrylamide; vinyl cyanide-based monomers such as (meth)acrylonitrile and α-chloroacrylonitrile; saturated aliphatic carboxylic acid vinyl ester monomers such as vinyl acetate and vinyl propionate; styrene-based monomers such as styrene, α-methylstyrene, and vinyltoluene; and the like.

These monomers may be used alone or in combination of two or more. In this description, "(meth) acrylate" means "acrylate or methacrylate".

As a polymerization method for the monomer mixture, a method commonly used by those skilled in the art can be employed. Examples of the polymerization method include bulk polymerization, solution polymerization, and a bulk-suspension two-stage polymerization method involving suspension polymerization after bulk polymerization, using a radical polymerization initiator.

A suitable specific example of the paint includes, for example, a hydroxyl group-containing acrylic resin disclosed in Non-Patent Literature 1, obtained by adding 4 PHR of azobisisobutyronitrile as an initiator to a monomer composition (hydroxyl value 150) of methyl methacrylate (weight ratio: 33), n-butyl acrylate (weight ratio: 15.9), 2-hydroxyethyl acrylate (weight ratio: 31.1), and styrene (weight ratio: 20), and polymerizing at 80°C using methyl ethyl ketone as a solvent (weight average molecular weight: 12500, number average molecular weight: 6430).

In the sample used in the first aspect of the present invention, it is preferable that the isocyanate curing agent and the paint are mixed such that the hydroxyl groups and isocyanate groups are equimolar, based on the monomer blending ratio of the hydroxyl group-containing acrylic resin and the manufacturer's analysis value of the isocyanate curing agent, as described in Non-Patent Literature 1.

The first aspect of the present invention preferably further includes a step of removing the cured product of the sample from the diamond prism after the infrared absorption spectrum measurement. In the first aspect of the present invention, the sample is brought into contact with the diamond prism to observe the curing process in order to prevent data from contaminants from entering the infrared absorption spectrum. For this reason, after the infrared absorption spectrum measurement, the cured product of the sample adheres to the surface of the diamond prism.

The cured product of the sample is removed, for example, by wiping the diamond prism with a cotton swab moistened with a small amount of solvent, followed by dry wiping. The solvent is not particularly limited as long as it is a solvent conventionally used for cleaning diamond prisms, and examples include methanol, ethanol, 2-propanol, N-hexane, toluene, tetrahydrofuran, ethyl acetate, and the like.

Hereinafter, the present invention will be described in more detail with reference to Experimental Examples, but the present invention is not limited thereto.

<Experimental Example 1>

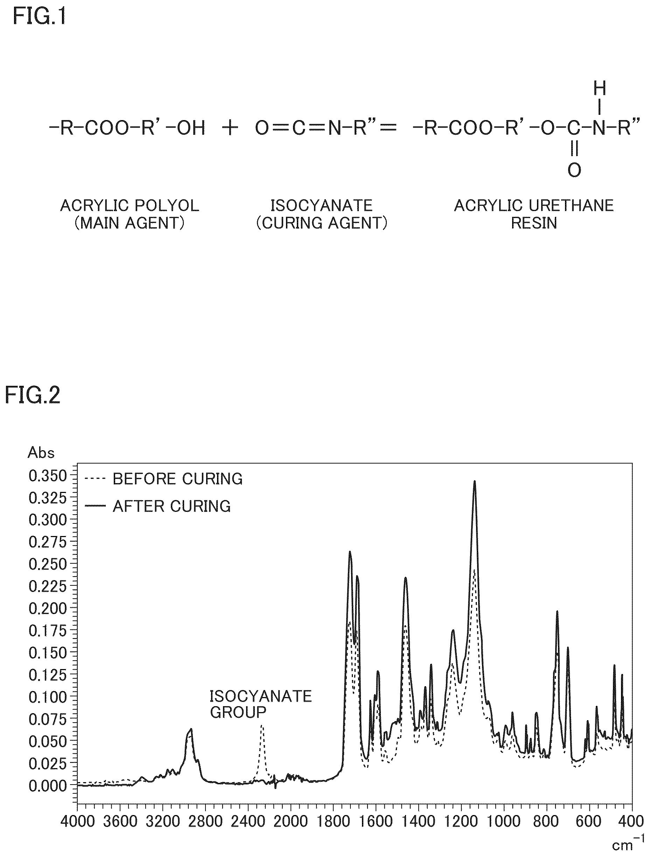

Using a Fourier Transform Infrared Spectrophotometer (FTIR) IRTracer-100 (trademark) (manufactured by Shimadzu Corporation) and a heating-type single-reflection attenuated total reflection measuring device MicromATR (manufactured by Czitech) equipped with a diamond prism, the thermal Curing reaction of a paint was measured in real time. In the thermal Curing reaction of the paint, the structure of the paint partially changes, and by focusing on the peak of the functional group derived from this changing structure, optimal curing conditions can be determined. In this Experimental Example, an acrylic urethane paint used for automobiles was measured. This paint uses an acrylic polyol as a main agent and an aliphatic polyisocyanate as a curing agent. When these two liquids are mixed, a polymerization reaction starts, forming urethane bonds (reaction formula is shown in FIG. 1). Acrylic urethane paint is a tough paint with weather resistance and gasoline resistance. Compared to other lacquer paints, it takes time to dry, but the drying time can be shortened by applying heat.

Measurements were performed under the following conditions.

Resolution: 4 cm$^{-1}$
Accumulation: 20
Wavenumber range: 4000 to 400 cm$^{-1}$
Apodization function: Happ-Genzel
Detector: DLATGS (Deuterated L-Alanine Triglycine Sulphate)

In MicromATR, measurements were performed while heating using a heating disk (the heating disk of MicromATR allows measurement in a temperature range from room temperature to 130°C).

A commercially available acrylic urethane paint (88 Panarock (manufactured by Rock Paint Co., Ltd.)) was used for the measurement. The main agent and the curing agent were mixed at a ratio of 10:1, stirred well, and then one drop of the paint was placed on the ATR diamond prism for measurement. FIG. 2 shows an overlay of the spectrum before curing, measured immediately after mixing the main agent and the curing agent, and the spectrum measured after leaving the mixture at room temperature for 3 days to completely cure. In the spectrum before curing, the isocyanate group contained in the curing agent can be confirmed around 2270 cm$^{-1}$, but after curing, the isocyanate group is not observed. This indicates that information regarding curing can be obtained by focusing on the isocyanate group.

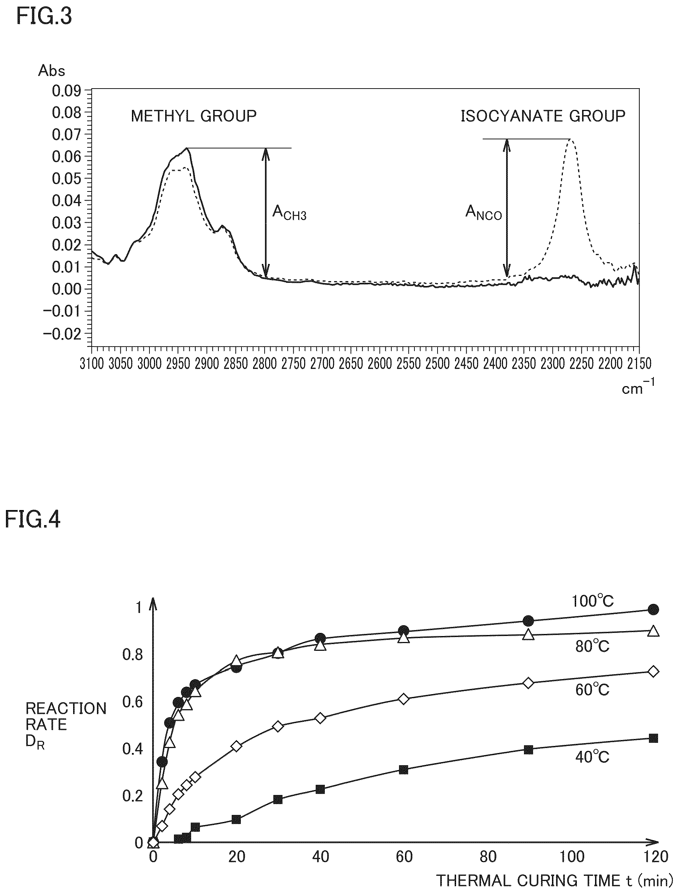

After mixing the main agent and curing agent of the paint, time-course measurements were performed for 2 hours at each constant temperature, and the reaction rate $D_R$ was calculated from the obtained spectra. Here, FIG. 3 is an enlarged spectrum of the portion from 3100 to 2150 cm$^{-1}$ in FIG. 2. As shown in FIG. 3, the reaction rate $D_R$ was calculated from the following formulas by calculating the peak intensity derived from the isocyanate group around 2270 cm$^{-1}$, based on the peak intensity ($A_{CH3}$) derived from the methyl group around 2940 cm$^{-1}$, which does not change before and after thermal Curing.

[Formula 1]

$$R(t) = A_{NCO}(t) / A_{CH3}(t)$$
$$D_R = 1 - R(t) / R(0)$$

t: thermal Curing time
$A_{NCO}(t)$: peak intensity around 2270 cm$^{-1}$ at thermal Curing time t
$A_{CH3}(t)$: peak intensity around 2940 cm$^{-1}$ at thermal Curing time t
R(t): peak intensity ratio of isocyanate group to methyl group at thermal Curing time t
$D_R$: reaction rate of polymerization reaction

FIG. 4 shows a plot of the calculated reaction rate $D_R$ and the thermal Curing time t. Since the reaction rate $D_R$ approaches 1 as thermal Curing progresses, it is possible to infer effective conditions for the thermal Curing reaction of the paint from FIG. 4.

As shown in FIG. 4, at set temperatures of 80°C and 100°C, thermal Curing progresses rapidly, reaching a reaction rate of about 0.8 within 30 minutes after the start of curing. From the viewpoint of $CO_2$ reduction, a thermal Curing temperature of 80°C is more suitable than 100°C. For reference, at set temperatures of 60°C and 40°C, the reaction rate was 0.7 or less even after 2 hours, indicating that more time is required for complete curing.

As described above, by measuring the thermal Curing reaction of the paint in real time using FTIR and a heating ATR accessory, it was found that for the acrylic urethane paint, thermal Curing progressed with increasing temperature, and no change in the thermal Curing reaction rate was observed at 80°C or higher. From this, it was inferred that 80°C is suitable as the thermal Curing temperature. By tracking the thermal Curing reaction in real time, it became possible to estimate the optimal thermal Curing temperature and time.

[Aspects]

It will be understood by those skilled in the art that the exemplary embodiments described above are specific examples of the following aspects.

(Item 1)

A method for in-situ observation of a curing state of a paint according to one aspect includes: a step of bringing a sample, which is a mixture of a curing agent and a paint, into contact with a surface of a diamond prism; a step of measuring an infrared absorption spectrum of the sample while controlling a temperature of the sample, and acquiring time-series data of an amount of a functional group possessed by the curing agent in the sample from the obtained infrared absorption spectrum; and a step of visualizing a relationship between time or temperature and the amount of the functional group possessed by the curing agent in the sample.

According to the method described in Item 1, data regarding the curing state of the paint can be acquired from the viewpoint of temperature and time during the paint curing process, thereby reducing the user's burden required to find the curing conditions for the paint to be evaluated.

(Item 2)

In the method according to Item 1, the step of measuring the infrared absorption spectrum of the sample while controlling the temperature of the sample, and acquiring time-series data of the amount of the functional group possessed by the curing agent in the sample from the obtained infrared absorption spectrum, includes both measuring the infrared absorption spectrum of the sample while maintaining the temperature of the sample constant, and measuring the infrared absorption spectrum of the sample while changing the temperature of the sample at a predetermined rate of change, and acquiring time-series data of the amount of the functional group possessed by the curing agent in the sample from the obtained infrared absorption spectrum.

According to the method described in Item 2, by including both measuring the infrared absorption spectrum of the sample while maintaining the temperature of the sample constant, and measuring the infrared absorption spectrum of the sample while changing the temperature of the sample at a predetermined rate of change, it becomes easier to visualize the relationship between time or temperature and the amount of the functional group possessed by the curing agent in the sample.

(Item 3)

The method according to Item 1, further includes a step of removing the cured product of the sample from the diamond prism after the infrared absorption spectrum measurement.

According to the method described in Item 3, by bringing the sample into direct contact with the diamond prism to measure the infrared absorption spectrum, data from contaminants do not enter the infrared absorption spectrum, as would be the case, for example, if a transparent sheet were interposed between the diamond prism and the sample.

(Item 4)

In the method according to any one of Items 1 to 3, the curing agent is an isocyanate curing agent, and the functional group possessed by the curing agent in the sample is an isocyanate group.

According to the method described in Item 4, the relationship between the infrared absorption spectrum of the isocyanate group and its curing temperature is known, facilitating analysis.

**Claims**

1.  A method for in-situ observation of a curing state of a paint, comprising: a step of bringing a sample, which is a mixture of a curing agent and a paint, into contact with a surface of a diamond prism; a step of measuring an infrared absorption spectrum of the sample while controlling a temperature of the sample, and acquiring time-series data of an amount of a functional group possessed by the curing agent in the sample from the obtained infrared absorption spectrum; and a step of visualizing a relationship between time or temperature and the amount of the functional group possessed by the curing agent in the sample.

2. The method according to Claim 1, wherein the step of measuring the infrared absorption spectrum of the sample while controlling the temperature of the sample, and acquiring time-series data of the amount of the functional group possessed by the curing agent in the sample from the obtained infrared absorption spectrum, comprises both measuring the infrared absorption spectrum of the sample while maintaining the temperature of the sample constant, and measuring the infrared absorption spectrum of the sample while changing the temperature of the sample at a predetermined rate of change, and acquiring time-series data of the amount of the functional group possessed by the curing agent in the sample from the obtained infrared absorption spectrum.

3. The method according to Claim 1, further comprising a step of removing a cured product of the sample from the diamond prism after the infrared absorption spectrum measurement.

4. The method according to any one of Claims 1 to 3, wherein the curing agent is an isocyanate curing agent, and the functional group possessed by the curing agent in the sample is an isocyanate group.

## FIG.1

$$-R-COO-R'-OH \;+\; O=C=N-R'' = \; -R-COO-R'-O-\underset{\underset{O}{\parallel}}{C}-\underset{H}{\overset{H}{N}}-R''$$

| ACRYLIC POLYOL | ISOCYANATE | ACRYLIC URETHANE |
|:---:|:---:|:---:|
| (MAIN AGENT) | (CURING AGENT) | RESIN |

## FIG.2

FIG.3

FIG.4

## INTERNATIONAL SEARCH REPORT

| International application No. |
| --- |
| **PCT/JP2024/018806** |

### A. CLASSIFICATION OF SUBJECT MATTER

**G01N 21/3577**(2014.01)i; **G01N 21/552**(2014.01)i; **G01N 33/32**(2006.01)i
FI: G01N21/3577; G01N21/552; G01N33/32

According to International Patent Classification (IPC) or to both national classification and IPC

### B. FIELDS SEARCHED

Minimum documentation searched (classification system followed by classification symbols)

G01N 21/00-21/01; G01N 21/17-21/61; G01N 33/00; G01N 33/32; G01N 33/44; G01J 3/42-3/433; C08G 18/00-18/87; C08L 75/00-75/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2007-248431 A (SEIKO EPSON CORPORATION) 27 September 2007 (2007-09-27) paragraphs [0020]-[0032], fig. 1-3 | 1-3 |
| Y | | 4 |
| Y | JP 2004-021814 A (KONICA MINOLTA HOLDINGS, INC.) 22 January 2004 (2004-01-22) paragraphs [0077]-[0078], [0082] | 4 |
| A | JP 2021-135093 A (NIDEC CORPORATION) 13 September 2021 (2021-09-13) paragraphs [0009]-[0023], fig. 1-6 | 1-4 |
| A | JP 2001-296243 A (SUMITOMO ELECTRIC INDUSTRIES, LTD.) 26 October 2001 (2001-10-26) paragraphs [0002]-[0006], fig. 3 | 1-4 |
| A | JP 2006-003196 A (CANON KABUSHIKI KAISHA) 05 January 2006 (2006-01-05) entire text, fig. 1-3 | 1-4 |

☑ Further documents are listed in the continuation of Box C.   ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **31 July 2024** | **13 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
| --- |
| **PCT/JP2024/018806** |

### C.    DOCUMENTS CONSIDERED TO BE RELEVANT

| Category* | Citation of document, with indication, where appropriate, of the relevant passages | Relevant to claim No. |
| --- | --- | --- |
| A | JP 2002-022648 A (MITSUBISHI RAYON CO., LTD.) 23 January 2002 (2002-01-23) entire text, fig. 1 | 1-4 |
| A | JP 2000-055806 A (FUJIKURA LTD.) 25 February 2000 (2000-02-25) entire text, fig. 1 | 1-4 |
| A | JP 08-062126 A (BAYER AKTIENGESELLSCHAFT) 08 March 1996 (1996-03-08) entire text, fig. 1 | 1-4 |

## INTERNATIONAL SEARCH REPORT
### Information on patent family members

International application No.

**PCT/JP2024/018806**

| Patent document cited in search report | | | Publication date (day/month/year) | Patent family member(s) | | | Publication date (day/month/year) |
|---|---|---|---|---|---|---|---|
| JP | 2007-248431 | A | 27 September 2007 | (Family: none) | | | |
| JP | 2004-021814 | A | 22 January 2004 | US paragraphs [0093]-[0095] KR CN | 2003/0234294 10-2004-0002583 1469310 | A1 A A | |
| JP | 2021-135093 | A | 13 September 2021 | (Family: none) | | | |
| JP | 2001-296243 | A | 26 October 2001 | (Family: none) | | | |
| JP | 2006-003196 | A | 05 January 2006 | (Family: none) | | | |
| JP | 2002-022648 | A | 23 January 2002 | (Family: none) | | | |
| JP | 2000-055806 | A | 25 February 2000 | (Family: none) | | | |
| JP | 08-062126 | A | 08 March 1996 | US EP | 5578827 694781 | A A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KANJI MORI**. Tracking Method of Crosslinking Density during Curing Process of Paint Film -Proportionality between Storage Modulus and Crosslinking Density. *J. Jpn. Soc. Colour Mater*, 2013, vol. 86 (4), 123-127

- **KANJI MORI**. Tracking Method of Crosslinking Density during Curing Process of Paint Film -Proportionality between Storage Modulus and Crosslinking Density. *J. Jpn. Soc. Colour Mater.*, 2013, vol. 86 (4), 123-127
- **MORIO TSUGE**. Application of Fourier Transform Infrared Spectroscopy to the Analysis of Thermal Curing Resins. *Thermal Curing Resins*, 1991, vol. 12 (4), 209-234